# EUROPEAN PATENT APPLICATION

(11) **EP 4 546 233 A1**
(43) Date of publication of application: **30.04.2025**
(21) Application number: 24206642.1
(22) Date of filing: 15.10.2024
(51) Int. Cl.: G06Q 10/0631, G06Q 10/0639, G06Q 50/04, A61B 3/11, A61B 3/113, G06F 3/01

(54) **QUALITY ASSURANCE PROCESS USING HUMAN RESOURCES TO IMPROVE THE EFFECTIVENESS OF THE QUALITY MANAGEMENT SYSTEM**

(30) Priority: 24.10.2023 HU 2300358
(71) Applicant: Antra ID GmbH, 82538 Geretsried (DE)
(72) Inventor: Révész, Andrea, 2045 Törökbálint (HU); Révész, László, 2045 Törökbálint (HU)
(74) Representative: Mészarosné Donusz, Katalin

(57) **Abstract**

The invention is a quality assurance procedure using human resources to improve the effectiveness of the quality management system where the viewing direction (1) of a worker representing the human resource at a given place of work in a situation facing a monitor (2) of a given phase of a manufacturing process is displayed. The worker's presence is perceived mechanically by cameras and/or lidars (3) on the basis of methods known per se, the viewing direction (1) of the worker's eyes (4) and the pupil size of the eyes (4) are determined and at given intervals time stamps are generated and stored containing this information. The system (7) includes at least one server (5) connected informatically to the monitor (2). The screen of the monitor (2) is devided into parts containing smaller blocks (6) according to a predefined pattern. Worker parameter sets are assigned to each block (6), where the worker parameter sets include at least the viewing direction (1) of the eyes (4), their time duration related to a given block (6) and pupil size. Expectation parameter sets are also assigned to each block (6), which include at least parameters corresponding to the worker parameter sets. The worker parameter sets and the expectation parameter sets related to each block (6) are compared one by block (6) by an application running on at least one server (5) with a given set of criteria, which consists the maximum permitted deviation of the worker and the expectation parameter sets and in case of exceeding the maximum permitted deviation an intervention is carried out by block (6) on the monitor (2) and in the system (7).

## Description

The subject matter of the present invention is a quality assurance procedure using human resources to improve the effectiveness of the quality management system where the viewing direction of a worker representing the human resource at a given place of work in a situation facing a monitor of a given phase of a manufacturing process is displayed.

The worker's presence is perceived mechanically by cameras and/or lidars on the basis of methods known per se, the viewing direction of the worker's eyes and the pupil size of the eyes are determined and at given intervals time stamps are generated and stored containing this information. The system includes at least one server connected informatically to the monitor.

From the state of art in respect of technology there are several procedures known for the operation of a quality system involving human resources where the worker representing the human resources verifies the result of a given process. Such as the product produced at the end of a manufacturing process where the worker verifies visually if the end product is correct or not.

The Japanese patent specification JP2019191990 describes a procedure to verify the operation of a production plant. According to an embodiment of the invention it has a risk assessment unit to assess the risk of equipment and groups of equipment in a process and quality assurance database and by using the various information stored in the maintenance and inspection results. Unit defining the operational mode and human resource information are stored in a human resource information database. Nevertheless this human resource information database does not provide real-time, online information about the concentration status of the worker participating in the control process.

The objective of the present invention is to be able to evaluate automatically the attention, effectiveness of the quality control worker, taking her/his personal data protection into account.

The invention is based on the realisation that nowadays - with cameras, lidars by methods known per se - the presence of the worker, the viewing direction of her/his eyes and the current pupil sizes of the eyes can also be determined. Such or similar devices are used among others in some virtual reality applications, games, simulators, for example in the case of helmet mounted devices. If the measured viewing results are compared with their expected counterpart, by forming their deviation the concentration state of the worker involved in the inspection and her/his work pattern can be qualified. By evaluating this data important parameters can be obtained in a given quality assurance system.

In its most general embodiment according to the invention, the process according to the introductory paragraph is that the screen of the monitor is devided into parts containing smaller blocks according to a predefined pattern. Worker parameter sets are assigned to each block, where the worker parameter sets include at least the viewing direction of the eyes, their time duration related to a given block and pupil size.

Expectation parameter sets are also assigned to each block, which include at least parameters corresponding to worker parameter sets. The worker parameter sets and the expectation parameter sets related to each blocks are compared one by block by an application running on at least one server with a given set of criteria, which consists the maximum permitted deviation of the worker and the expectation parameter sets and in case of exceeding the maximum permitted deviation an intervention is carried out by block on the monitor and in the system.

Some further embodiments of the invention are defined by the attached sub-claims.

In the following, the invention is presented in more detail by means of diagrams, where the Figure 1 displays a worker involved in the inspection with a dedicated control panel, Figure 2 displays the monitor of the control panel according to 1. Figure with a given block layout, Figures 3/a., 3/b. and 3/c. together form a flow chart, which illustrates the procedure's steps.

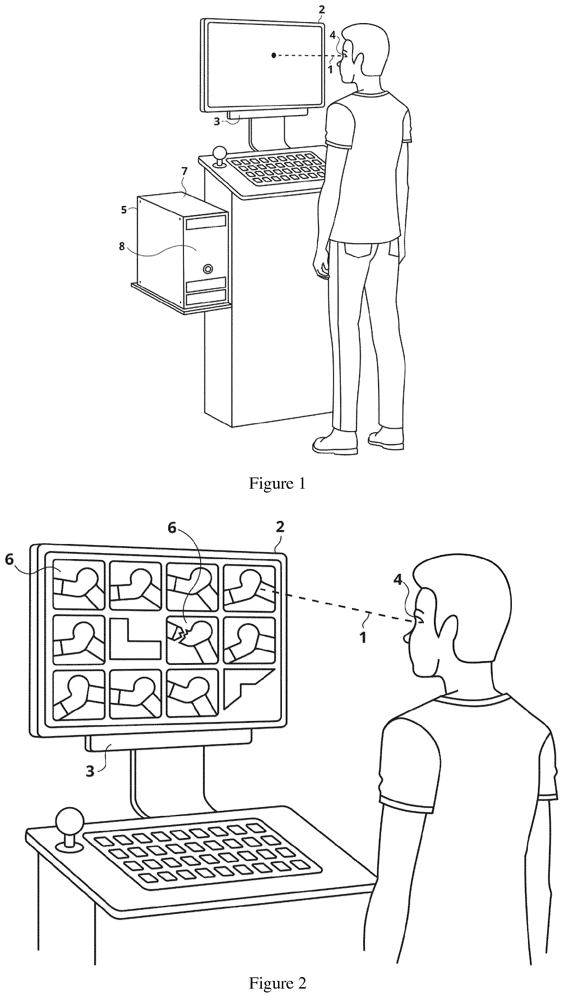

Figure 1 shows a worker involved in the inspection with a dedicated control panel, whose eyes 4 are directed to a monitor 2 at a given workplace in front of a monitor 2. The monitor 2 represents a given phase of a manufacturing process, e.g. completion phase. The worker's eyes 4 are observed by cameras and/or lidars 3 automatically by machine, using methods known per se, which for this reason will not be described here. On this basis the presence of the worker, the viewing direction 1 of her/his eyes 4 and the pupil sizes of the eyes 4 are also determined and at given intervals time stamps are generated and stored containing this information. The panel or other part of the system 7 contain at least one server 5 connected informatically to the monitor 2, which can also use a neural network 8, if applicable.

Figure 2 displays the control panel according to Figure 1 with its content with a given block layout. The monitor 2 is diveded into parts containing smaller blocks 6 according to a predefined pattern, the worker parameter sets are assigned to each blocks 6, where the worker parameter sets include at least the viewing direction 1 of the eyes 4, their time duration related to a given block 6 and pupil size. Expectation parameter sets are also assigned to each blocks 6, which include at least parameters corresponding to worker parameter sets.

The worker parameter sets and the expectation parameter sets related to each blocks 6 are compared one by block 6 by an application running on at least one server 5 with a given set of criteria, which consists the maximum permitted deviation of the worker and the expectation parameter sets and in case of exceeding the maximum permitted deviation an intervention is carried out by block on the screen and in the system. A maximum permitted deviation is defined and if these are exceeded an intervention is carried out by block 6 on the monitor 2 and in the system 7. An application running on the latter may use a 8 neural network. An intervention can be e.g. the visual highlight of the relevant blocks 6, e.g. flashing and/or colouring of the relevant blocks 6, which draws the worker's attention to a greater attention. The intervention can also be combined with an additional acoustic signal.

For a given embodiment the worker, the product and the work process can be assigned - proving the performance of the work and the quality of the products - for a given work process by a related digital validation certificate.

The procedure can also contain the statistical data analysis of the relevant blocks 6.

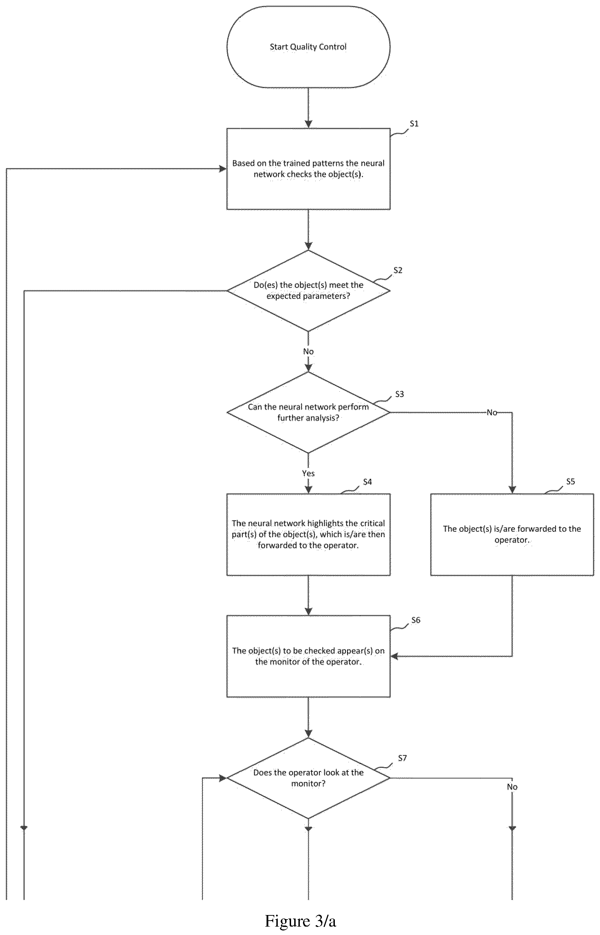

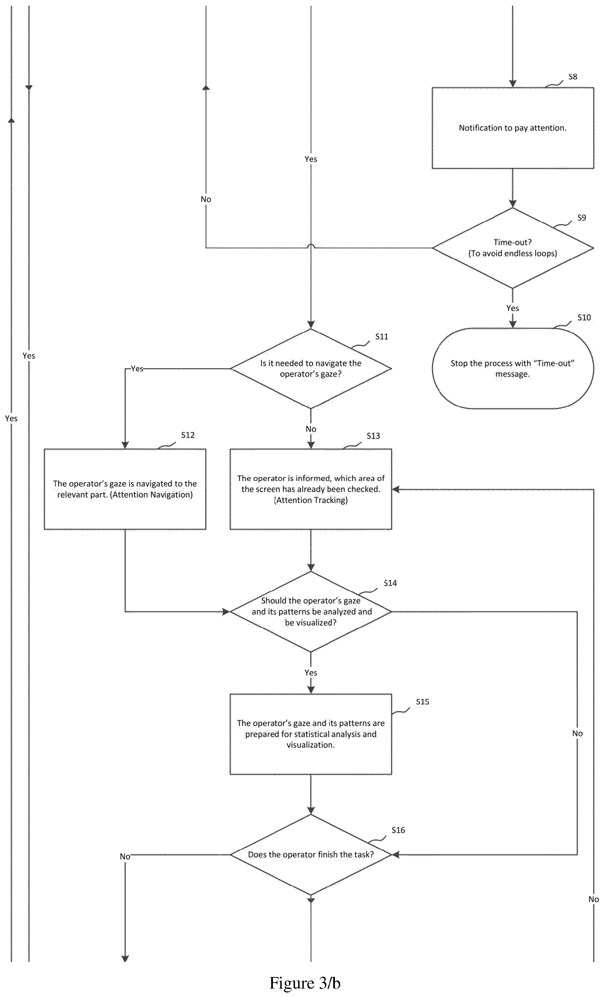

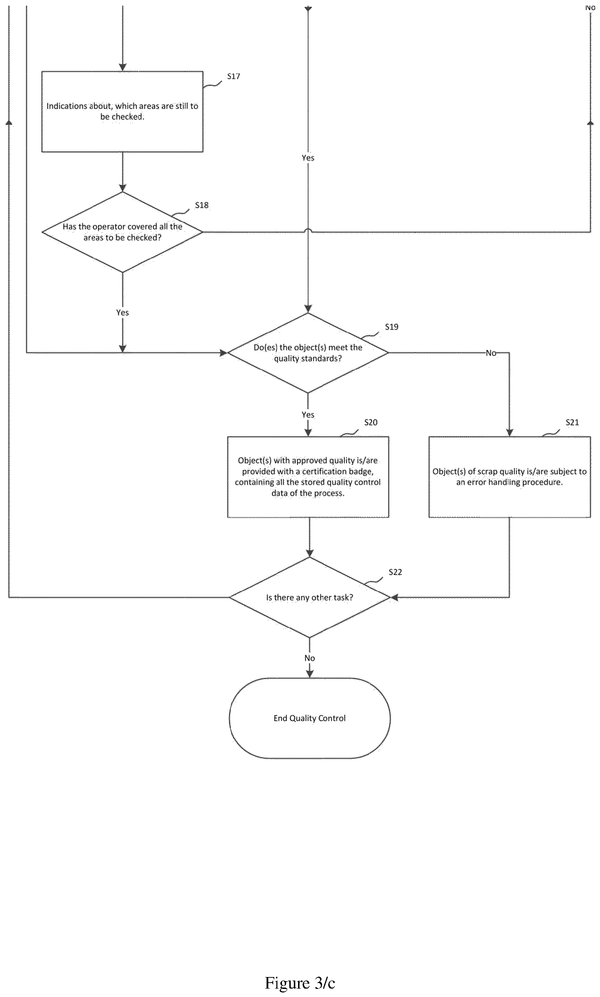

The steps of the procedure are described below with reference to Figures 3/a-3/b-3/c. This example also assumes the use of a neural network 8.

First, in step S1 the neural network 8 examines the object(s) so the blocks 6 embodying them on the basis of learned patterns, using methods known in the technology per se.

In step S2 expectation parameter sets are assigned to each block 6, they are compared to the worker parameter sets. An example of this is the following: if a predefined percentage (e.g. 80%) of the worker parameters is corresponding to the expectation parameters based on samples fed into the system 7, then the process proceeds with step S19 and it means the system 7 full automation.

In step S3 it is examined if the neural network 8 can make further analysis? If yes, in step S4 the neural network 8 highlights the critical area of the object(s) and transmits it to the operator.

If not, in step S5 the object(s) are forwarded to the operator, i.e. to worker.

In step S6 one or more objects to be inspected are displayed on the monitor 2 at the operator.

In step S7 the system 7 examines if the operator looks at the monitor 2 or not.

If not, in step S8 it gives a signal to pay attention. This can be visual and/or acoustic signal. In step S9 it is examined if there is a time-out in order to avoid an infinite loop. If there is a time-out, in step S9 a signal is given to raise awareness and in step S10 the work process is stopped with a "Time-out" message.

In step S11 it is examined if there is a need for navigating the operator's attention?

If yes, in step S12 the operator's attention is navigated to the relevant part.

If not, in step S13 the operator is informed which area of the screen has already been checked.

In step S14 it is examined if there is a need for analyzing and visualizing the operator's eye movement and its pattern.

If yes, in step S15 the operator's eye movement and its pattern are prepared for statistical analysis and visualization.

If not, in step S16 it is examined if the operator has done her/his job? If not, in step S17 a signal is given which area has been left out and in step S18 it is examined if the operator has covered the total area to be checked or not.

If not, the process restarts. If yes, in step S19 a data sheet is generated containing all relevant inspection data of the work process and stored in the system 7 and it is examined if the objects meet the quality standards. If yes, in step S20 a certification badge is given to the objects with approved quality by the system 7. If not, step S21 comes, in which the objects identified as scrap are indicated and an error handling procedure is initiated.

In step S22 it is examined if there is an additional task? If yes, the process restarts, if not the process ends.

The following is an example of this: at the end of a production line pictures of the cable harnesses and connectors of the electronic components manufactured on it are inspected by neural network and if necessary they are forwarded to the worker (operator) to monitor 2. If cameras and/or lidars 3 do not see the eyes 4 of the worker, i.e. 15 seconds (expectation parameter), the system 7 gives a visual and/or acoustic signal to the worker to pay attention and look at the monitor 2. If according to the set of criteria a given block has to be inspected e.g. not less than 2 seconds but not more than 10 seconds (expectation parameter) for proper quality control but from the data obtained from the viewing direction 1 of the worker (worker parameter) it seems that less time has passed than that when inspecting the block, then the viewing direction 1 of the worker is (re)-navigated to the given area by flashing the block and it is asked to check the block more carefully. If a block has been inspected in at least 2 seconds but no more than 10 seconds (worker parameter), the given block is faded away. If the viewing direction 1 related to the given block exceeds the 10 seconds (worker parameter), the system 7 gives visual and/or acoustic signal to the worker to raise awareness.

The entire process is only illustrative, other equivalent solutions can also be considered.

The advantage of the invention is that it complements automatic quality control solutions with the possibility of operating workstations with improved efficiency using human resources, workers and operators.

### List of references

- 1: Viewing direction
- 2: Monitor
- 3: Cameras and/or Lidars
- 4: Eyes
- 5: Server
- 6: Blocks
- 7: System
- 8: Neural network

## Claims

1. A quality assurance procedure using human resources to improve the effectiveness of the quality management system where the viewing direction (1) of a worker representing the human resource at a given place of work in a situation facing a monitor (2) of a given phase of a manufacturing process is displayed,the worker's presence is perceived mechanically by cameras and/or lidars (3) on the basis of methods known per se, the viewing direction (1) of the worker's eyes (4) and the pupil size of the eyes (4) are determined and at given intervals time stamps are generated and stored containing this information, the system (7) includes at least one server (5) connected informatically to the monitor (2),
**characterised in that**
the screen of the monitor (2) is devided into parts containing smaller blocks (6) according to a predefined pattern, worker parameter sets are assigned to each block (6), where the worker parameter sets include at least the viewing direction (1) of the eyes (4), their time duration related to a given block (6) and pupil size,
expectation parameter sets are also assigned to each block (6), which include at least parameters corresponding to the worker parameter sets,
the worker parameter sets and the expectation parameter sets related to each blocks (6) are compared one by block (6) by an application running on at least one server (5) with a given set of criteria, which consists the maximum permitted deviation of the worker and the expectation parameter sets
and in case of exceeding the maximum permitted deviation an intervention is carried out by block (6) on the monitor (2) and in the system (7).

2. The method according to claim 1, **characterised in that** the application also makes use of a neural network (8).

3. The method according to any one of claims 1 to 2, **characterized in that** the intervention comprises visually highlighting the respective blocks (6).

4. The method according to claim 3, **characterised in that** the visual highlighting comprises flashing and/or colouring relevant blocks (6).

5. The method according to any one of claims 1 to 4, **characterized in that** the intervention is further coupled with an additional acoustic signal.

6. The method according to any one of claims 1 to 5, **characterized in that** the worker, the product and the work process are provided with a digital certification badge associated with a given work process, certifying the performance of the work and the quality of the products.

7. A method according to any one of claims 1 to 6, **characterised in that** it comprises statistical data analysis of the relevant blocks (6).
